(19) Europäisches Patentamt
European Patent Office
Office européen des brevets

(11) **EP 4 649 882 A1**

(12) **EUROPEAN PATENT APPLICATION**
published in accordance with Art. 153(4) EPC

(43) Date of publication:
**19.11.2025 Bulletin 2025/47**

(21) Application number: **23916202.7**

(22) Date of filing: **13.12.2023**

(51) International Patent Classification (IPC):
***A61B 1/045*** (2006.01)

(52) Cooperative Patent Classification (CPC):
**A61B 1/045**

(86) International application number:
**PCT/JP2023/044619**

(87) International publication number:
**WO 2024/150588 (18.07.2024 Gazette 2024/29)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.01.2023 JP 2023002336**

(71) Applicant: **HOYA CORPORATION**
**Shinjuku-ku**
**Tokyo 160-8347 (JP)**

(72) Inventor: **MAKINO Takao**
**Tokyo 160-8347 (JP)**

(74) Representative: **Lucke, Andreas**
**Boehmert & Boehmert**
**Anwaltspartnerschaft mbB**
**Pettenkoferstrasse 22**
**80336 München (DE)**

(54) **PROCESSOR FOR ELECTRONIC ENDOSCOPES, AND ELECTRONIC ENDOSCOPIC SYSTEM**

(57)  In an aspect of the present invention, an aspect of the present invention is a processor for an electronic endoscope that acquires a captured image of a living tissue and performs image processing. The processor for an electronic endoscope includes an enhancement calculation unit (27) for the captured image of the living tissue. The enhancement calculation unit (27) includes: an edge detection unit (272) that detects an edge component for each of pixels of the captured image of the living tissue; an edge component correction unit (273) that corrects the edge component of each of the pixels detected by the edge detection unit (272) with reference to threshold setting data in which a threshold of the edge component in accordance with a luminance value is set; and an enhancement processing unit (274) that performs contour enhancement processing on the captured image based on the edge component corrected by the edge component correction unit (273).

*FIG. 2*

# Description

Technical Field

The present invention relates to a processor for an electronic endoscope that acquires a captured image of a living tissue and performs image processing on the captured image, and an electronic endoscope system.

Background Art

An electronic endoscope device is used for observation and treatment of a living tissue inside a human body. Enhancement processing for making a specific element of the living tissue noticeable from a captured image obtained by capturing the living tissue using the electronic endoscope device is performed on the captured image, and the resultant is displayed on a display.

For example, JP 2009-21905 A discloses a contour enhancement device that detects an edge component of each pixel of an input luminance signal and adds a weight to the detected edge component. The contour enhancement device is configured such that a relatively large weight is added to an edge component related to a pixel in which a luminance value of the input luminance signal is at a middle level, and a relatively small weight is added to an edge component related to a pixel in which a luminance value of the input luminance signal is at a low level or at a high level, whereby a contour of an object can be appropriately emphasized while preventing occurrence of a large undershoot (cat's eye) around the contour.

Summary of Invention

Technical Problem

In general, since a living tissue to be observed often has a portion covered with a mucous membrane, specular reflection is likely to occur, and a captured image may include a whitened portion.

In a contour enhancement method described in JP 2009-21905 A, since a relatively lower weight is applied to a high-luminance portion of an image, contour enhancement of the high-luminance portion is suppressed. Therefore, although occurrence of a large undershoot in the whitened portion can be prevented, the contour enhancement is suppressed even in the high-luminance portion other than the whitened portion in the captured image, so that there is a problem that a blurred image is likely to occur.

Therefore, an object of the present invention is to suppress occurrence of a large undershoot and appropriately emphasize a portion to be originally emphasized when contour enhancement processing is performed on a captured image in a processor for an electronic endoscope.

Solution to Problem

An aspect of the present disclosure is a processor for an electronic endoscope that acquires a captured image of a living tissue and performs image processing. The processor for an electronic endoscope includes:

an edge detection unit that detects an edge component for each of pixels of the captured image of the living tissue;
an edge component correction unit that corrects the edge component of each of the pixels detected by the edge detection unit with reference to threshold setting data in which a threshold of the edge component in accordance with a luminance value is set; and
an enhancement processing unit that performs contour enhancement processing on the captured image based on the edge component corrected by the edge component correction unit.

In the threshold setting data, the threshold may be set such that the threshold of the edge component increases as the luminance value increases.

In a case where the edge component detected by the edge detection unit exceeds the threshold, the edge component correction unit may correct the edge component based on a parameter set to decrease a portion exceeding the threshold in the edge component. In such a case, the parameter is set such that the portion exceeding the threshold decreases as an intensity of enhancement of the contour enhancement processing increases.

The processor for an electronic endoscope preferably includes an intensity changing unit that changes the intensity of enhancement of the contour enhancement processing in accordance with an operation of a user. When the intensity of enhancement of the contour enhancement processing is changed, the edge component correction unit adjusts the parameter based on the changed intensity.

The enhancement processing unit may perform the contour enhancement processing according to a contour enhancement processing method selected in accordance with an operation of the user from among a plurality of the contour enhancement processing methods. In such a case, the plurality of contour enhancement processing methods are associated with different pieces of the threshold setting data, respectively.

The edge component correction unit corrects the edge component with reference to the threshold setting data associated with the selected contour enhancement processing method.

The enhancement processing unit may perform the contour enhancement processing according to a contour enhancement processing method selected in accordance with an operation of the user from among a plurality of the contour enhancement processing methods. In such a case, the plurality of contour enhancement processing methods are associated with the parameters different from each other.

The edge component correction unit corrects the edge component with reference to the parameter associated with the selected contour enhancement processing method.

The processor for an electronic endoscope may further include a light source unit that generates, as illumination light for illuminating the living tissue, either first illumination light in a first wavelength band or second illumination light in a second wavelength band different from the first wavelength band. In such a case, the first illumination light and the second illumination light are associated with different pieces of the threshold setting data.

The edge component correction unit corrects the edge component with reference to the threshold setting data associated with the illumination light generated by the light source unit out of the first illumination and the second illumination light.

The processor for an electronic endoscope may further include a light source unit that generates, as illumination light for illuminating the living tissue, either first illumination light in a first wavelength band or second illumination light in a second wavelength band different from the first wavelength band. In such a case, the first illumination light and the second illumination light are associated with the parameters different from each other.

The edge component correction unit corrects the edge component with reference to the parameter associated with the illumination light generated by the light source unit out of the first illumination and the second illumination light.

Another aspect of the present disclosure is an endoscope system including: the processor for an endoscope; and

an endoscope connected to the processor for an endoscope and including an image sensor that captures the living tissue.

Advantageous Effects of Invention

According to the processor for an electronic endoscope and the electronic endoscope system described above, when the contour enhancement processing is performed on the captured image in the processor for an electronic endoscope, it is possible to appropriately emphasize the portion to be originally emphasized while suppressing the occurrence of the large undershoot.

Brief Description of Drawings

Fig. 1 is a block diagram illustrating an example of a configuration of an electronic endoscope system according to one embodiment.
Fig. 2 is a block diagram illustrating a configuration of an enhancement calculation unit that performs edge enhancement of an image signal.
Fig. 3 is a view illustrating processing content of an edge component correction unit as an example.
Fig. 4 is a view illustrating an example of threshold setting data set in the enhancement calculation unit.
Fig. 5 is a flowchart illustrating details of processing of the enhancement calculation unit.
Fig. 6 is a view illustrating image examples in which conventional edge enhancement is compared with the edge enhancement according to the embodiment.

Description of Embodiments

An electronic endoscope system according to the present embodiment will be described below in detail with reference to the drawings.

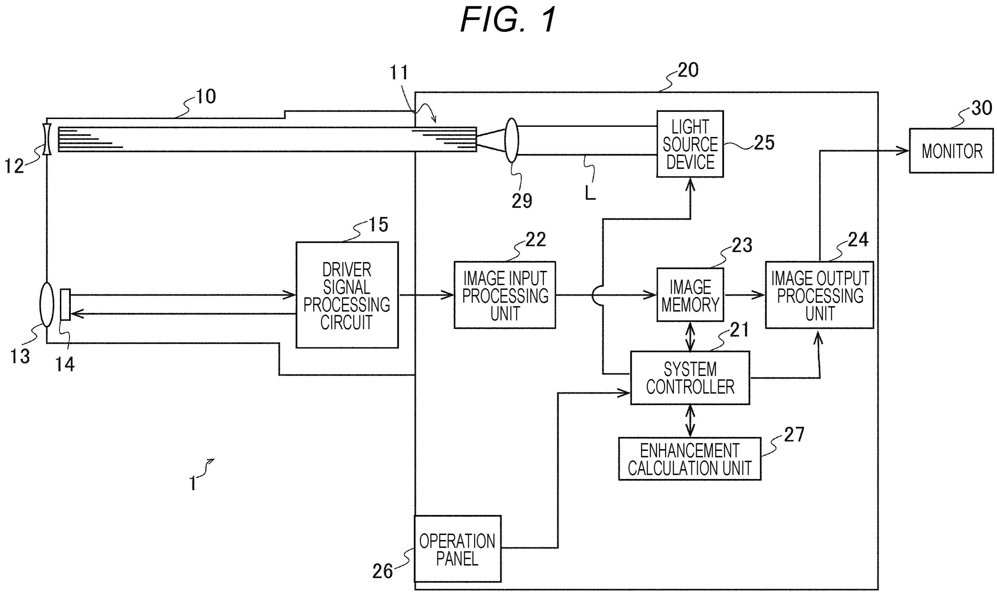

Fig. 1 is a block diagram illustrating an example of a configuration of an electronic endoscope system 1 of the present embodiment. As illustrated in Fig. 1, the electronic endoscope system 1 is a system specialized for medical use, and includes an electronic scope (endoscope) 10, a processor 20, and a monitor 30.

The processor 20 includes a system controller 21. The system controller 21 executes various programs and integrally controls the entire electronic endoscope system 1. In addition, the system controller 21 is connected to the operation panel 26. The system controller 21 changes operations of the electronic endoscope system 1 and a parameter for each of the operations in accordance with an instruction of an operator (observer) input to the operation panel 26. The system controller 21 outputs a clock pulse for adjusting an operation timing of each unit to the corresponding circuit in the electronic endoscope system 1.

The processor 20 includes a light source device 25 (an example of a light source unit). The light source device 25 emits illumination light L for illuminating an object such as a living tissue in a body cavity. The illumination light L includes white light, pseudo white light, or special light. According to one embodiment, it is preferable that the light source device 25 select one of a mode of constantly emitting the white light or the pseudo white light as the illumination light L and a mode of alternately emitting the white light or the pseudo white light and the special light as the illumination light L, and emit the white light, the pseudo white light, or the special light based on the selected mode. The white light is light having a flat spectral intensity distribution in a visible light band, and the pseudo white light is light which is a mixture of light of a plurality of wavelength bands and has non-flat spectral intensity distribution. The special light is light of a narrow wavelength band, such as blue or green, in the visible light band. The light of the blue or green wavelength band is used at the time of enhancing a specific portion of the living tissue and observing the portion. The illumination light L emitted from the light source device 25 is condensed onto an incident end face of a light carrying bundle (LCB) 11 by a condenser lens 29, and enters the LCB 11.

A light source of the light source device 25 is not limited, and is, for example, an LED, a laser diode, a high-luminance lamp (for example, a xenon lamp, a metal halide lamp, a mercury lamp, or a halogen lamp), or the like.

The illumination light L entering the LCB 11 propagates through the LCB 11. The illumination light L propagating through the LCB 11 is emitted from an exit end face of the LCB 11 disposed at a distal tip of the electronic scope 10, and irradiates the object via a light distribution lens 12. Return light from the object illuminated with the illumination light L from the light distribution lens 12 forms an optical image on a light receiving surface of a solid-state image sensor 14 via an objective lens 13.

The solid-state image sensor 14 is, for example, a single-plate color charge coupled device (CCD) image sensor having a Bayer pixel arrangement. The solid-state image sensor 14 accumulates an optical image formed by each of pixels on the light receiving surface as charges corresponding to the amount of light, and generates and outputs image signals of red (R), green (G), and blue (B). Note that the solid-state image sensor 14 is not limited to a CCD image sensor, and may be replaced with a complementary metal oxide semiconductor (CMOS) image sensor or other types of imaging devices. In addition, the solid-state image sensor 14 may include a complementary color filter.

A driver signal processing circuit 15 is provided in a connection portion of the electronic scope 10.

The system controller 21 supplies a clock pulse to the driver signal processing circuit 15. The driver signal processing circuit 15 controls driving of the solid-state image sensor 14 at a timing synchronized with a frame rate of a video image processed by the processor 20 in accordance with the clock pulse supplied from the system controller 21. An image signal of the object is input to the driver signal processing circuit 15 from the solid-state image sensor 14 in a predetermined frame cycle. The frame cycle is, for example, 1/30 seconds or 1/60 seconds. The driver signal processing circuit 15 performs predetermined processing including A/D conversion on the image signal input from the solid-state image sensor 14 and outputs the processed image signal to an image input processing unit 22 of the processor 20.

The image input processing unit 22 performs predetermined signal processing such as noise removal processing, demosaic processing, and matrix calculation on the image signal transmitted from the driver signal processing circuit 15.

An image memory 23 is a memory that buffers the image signal transmitted from the image input processing unit 22 in units of frames. Images of the respective frames accumulated in the image memory 23 are sequentially subjected to edge enhancement processing in an enhancement calculation unit 27 under the control of the system controller 21. Details of the enhancement calculation unit 27 will be described later. The image signal subjected to the edge enhancement processing is output to an image output processing unit 24 according to timing control by the system controller 21.

The image output processing unit 24 processes the image signal subjected to the edge enhancement by the enhancement calculation unit 27 in units of frames to generate screen data for monitor display, and converts the generated screen data for monitor display into a predetermined video format signal. The converted video format signal is output to the monitor 303. As a result, an image of the object is displayed on a display screen of the monitor 30.

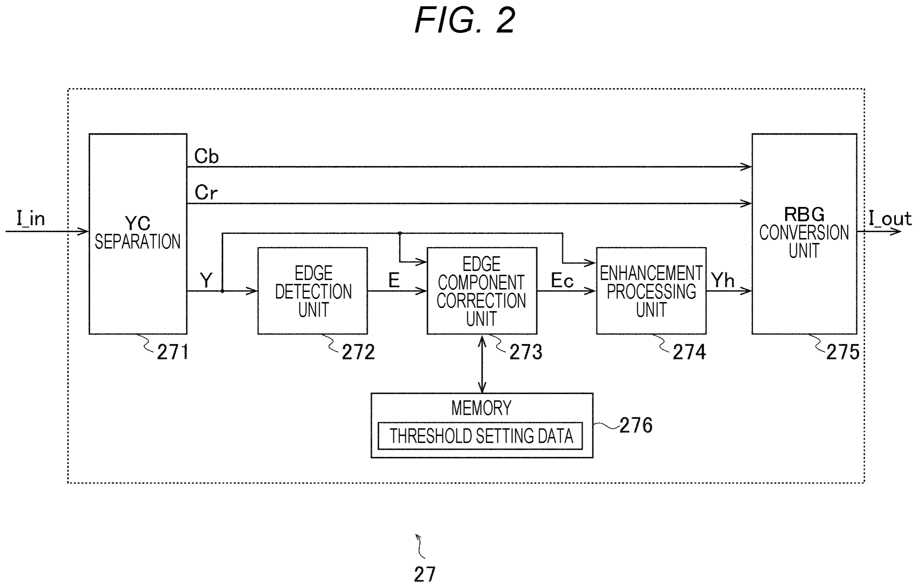

Next, a configuration of the enhancement calculation unit 27 will be described with reference to Fig. 2.

In Fig. 2, the enhancement calculation unit 27 performs edge enhancement processing on an input image I_in (image signal accumulated in the image memory 23) transmitted from the system controller 21, and generates an output image I_out subjected to which the edge enhancement has been applied.

As illustrated in Fig. 2, the enhancement calculation unit 27 includes a YC separation unit 271, an edge detection unit 272, an edge component correction unit 273, an enhancement processing unit 274, an RBG conversion unit 275, and a memory 276.

The input image I_in is an RGB signal. The YC separation unit 271 separates (converts) the RGB signal of the input image I_in into a luminance signal Y and chrominance signals Cb and Cr.

The edge detection unit 272 detects an edge component in units of pixels from the luminance signal Y obtained by the YC separation unit 271. For the detection of the edge component, for example, a known spatial filter such as a Laplacian filter or a Sobel filter can be adopted.

The edge component correction unit 273 corrects ah edge component E detected by the edge detection unit 272. The edge component E is corrected in order to appropriately emphasize a portion to be originally emphasized while suppressing occurrence of a large undershoot. For correction of the edge component E, the edge component correction unit 273 accesses the threshold setting data. The threshold setting data is stored in the memory 276 which is a nonvolatile memory. In the threshold setting data, a threshold of an edge component is set with respect to a luminance value indicated by the luminance signal Y.

In one embodiment, the edge component correction unit 273 corrects the edge component E of each pixel detected by the edge detection unit 272 with reference to the threshold setting data. For example, the edge component E of each pixel is limited to be equal to or less than the threshold set in accordance with the luminance value of each pixel.

In general, in an object covered with a mucous membrane such as a stomach or a large intestine, a high-luminance whitened portion is generated by specular reflection of illumination light. Conventionally, when edge enhancement is applied to such a whitened portion, unnatural emphasis (edge undershoot) in which the whitened portion is bordered in black is likely to occur. On the other hand, if the edge component E is limited to be equal to or less than the threshold set in advance in accordance with the luminance value, there is an advantage that this edge undershoot is less likely to occur.

It is preferable that the threshold setting data be set so as not to cause the edge undershoot of the whitened portion and so as not to suppress emphasis of a portion to be normally observed (for example, a blood vessel portion) other than the whitened portion in an image. In a case where the edge component E is less than the threshold according to the luminance value, the edge component E is not decreased, and thus the enhancement performance is not deteriorated. Therefore, it is preferable not to make the threshold too low so as not to deteriorate the enhancement performance of the portion to be normally observed other than the whitened portion.

The enhancement processing unit 274 combines the luminance signal Y generated by the YC separation unit 271 and an edge component Ec, which is a component obtained by correcting the edge component E by the edge component correction unit 273, to generate a luminance signal Yh subjected to contour enhancement processing (edge enhancement). At this time, in a case where an intensity of the enhancement is changed in accordance with an operation of a user, the enhanced luminance signal Yh is generated by adding the luminance signal Y and the edge component Ec multiplied by a coefficient that increases as the intensity increases.

The RBG conversion unit 275 converts the chrominance signals Cb and Cr separated by the YC separation unit 271 and the luminance signal Yh obtained by the enhancement processing unit 274 into an RGB signal to generate the output image I_out.

In one embodiment, in a case where the edge component E detected by the edge detection unit 272 exceeds the threshold set by the threshold setting data, the edge component correction unit 273 corrects the edge component E based on a parameter set to decrease a portion exceeding the threshold in the edge component E. That is, since there is a possibility that the portion exceeding the threshold in the edge component E is a whitened portion, the edge component E in this portion is decreased. Here, the parameter is set such that the portion exceeding the threshold decreases as the intensity of edge enhancement by the spatial filter for edge detection applied in the edge detection unit 272 increases.

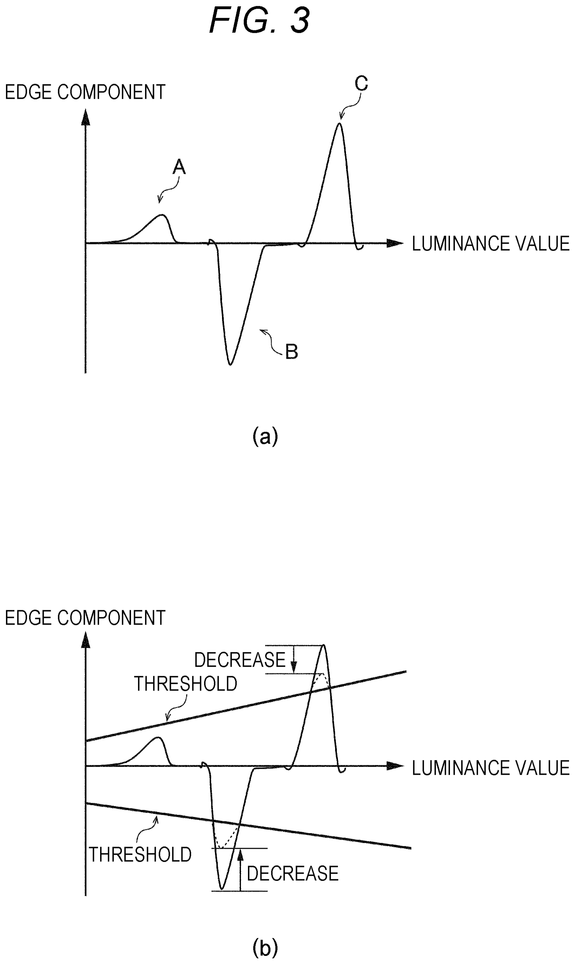

Fig. 3 illustrates processing content of the edge component correction unit 273 as an example of one embodiment.

Fig. 3(a) is a view illustrating an example of distribution of luminances and edge components, detected by the edge detection unit 272, of a plurality of pixels included in a partial region of an input image. In Fig.

3(a), it is assumed that a portion A is a portion to be normally observed other than a whitened portion, and portions B and C are whitened portions.

In addition, it is assumed that the threshold illustrated in Fig. 3(b) is set by the threshold setting data with respect to the example of Fig. 3(a). At this time, in a case where an edge component detected for a certain pixel is equal to or less than the threshold set in accordance with the luminance value, the edge component is not substantially corrected. On the other hand, in a case where an edge component detected for a certain pixel is more than the threshold set in accordance with the luminance value, the edge component is corrected based on a parameter set to decrease a portion exceeding the threshold in the detected edge component. In Fig. 3(b), the edge component after the decrease is indicated by a dotted line.

In the example of Fig. 3(a), the portion A is not corrected, but the portions B and C are more than the threshold set in accordance with the luminance value and thus are corrected such that portions exceeding the threshold decrease.

Here, when a decrease rate of the portion exceeding the threshold is r (incidentally, $0 \leq r < 1$), the parameter is set so as to satisfy the following Formula (1). Here, f is a function of an intensity S of edge enhancement, and the parameter is set in the function f defined here.

$$r = f(S) \ldots (1)$$

In one embodiment, the function is defined as $f = \alpha \times S$. Here, a parameter $\alpha$ is set such that the decrease rate r becomes a specific value of $0 \leq r < 1$ in accordance with the intensity S of edge enhancement. For example, in a case where the intensity S of edge enhancement is set to be variable among six levels of 1 to 6, the decrease rate r is set to 0.25 regardless of the intensity S of edge enhancement by setting values of the parameter $\alpha$ when the intensity S of edge enhancement is 1 to 6 to 1/4, 1/8, 1/12, 1/16, 1/20, and 1/24, respectively. Note that it is not necessary to make the decrease rate r constant regardless of the intensity S of edge enhancement, and the function f may be defined such that the decrease rate r changes in accordance with the intensity S of edge enhancement as long as the decrease rate r is in the range of $0 \leq r < 1$.

When the edge component E is corrected based on the parameter set to decrease the portion exceeding the threshold in the edge component E detected by the edge detection unit 272, it is possible to suppress the edge undershoot of the whitened portion even in a case where the intensity of edge enhancement is increased (that is, in a case where enhancement is strongly applied).

Note that the function f in Formula (1) is not limited to one using a parameter that linearly changes in accordance with the magnitude of the intensity S of edge enhancement as defined by f = α × S, and may be one using a parameter that non-linearly changes in accordance with the magnitude of the intensity S of edge enhancement. In addition, the function f of the intensity S of edge enhancement may differ depending on whether the edge component is positive or negative.

The enhancement calculation unit 27 of one embodiment is configured such that the intensity of edge enhancement is set to be changeable, and the observer of the monitor 30 can set a desired intensity from among a plurality of intensities by operating the operation panel 26. In such a case, as described above, the enhancement processing unit 274 adds the luminance signal Y generated by the YC separation unit 271 and an edge component obtained by multiplying the corrected edge component Ec by the coefficient that increases as the intensity increases, to generate the enhanced luminance signal Yh. That is, the enhancement processing unit 274 functions as an intensity changing unit that changes the intensity of edge enhancement in accordance with the operation of the user. In a case where the intensity of enhancement of the edge enhancement processing is changed, the edge component correction unit 273 adjusts the parameter based on the changed intensity, thereby performing edge enhancement after setting an optimum parameter in accordance with the intensity of edge enhancement selected by the user.

In one embodiment, in the threshold setting data, the threshold is preferably set such that the threshold of the edge component increases as the luminance value increases. This is to suppress the edge undershoot of the whitened portion that becomes more conspicuous as the luminance value increases.

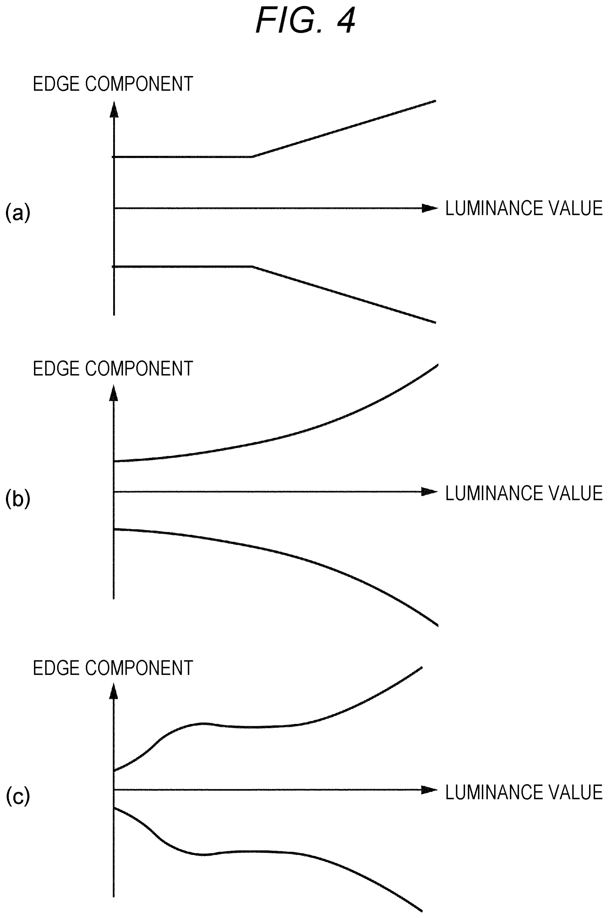

Although a case where the threshold of the edge component linearly increases with respect to the luminance value is illustrated in the example illustrated in Fig. 3(b), the present invention is not limited thereto. Fig. 4 illustrates an example of another threshold setting data. As illustrated in Figs. 4(a) to 4(c), a threshold of an edge component with respect to a luminance value may be set non-linearly with respect to the luminance value, or there may be a region in which the threshold with respect to the luminance value is partially constant.

As illustrated in Fig. 3(b), in a case where the threshold of the edge component is linearly increased with respect to the luminance value, it is preferable to determine the slope in consideration of how much edge component is present in a region where the luminance value is high to make the edge undershoot visible in an image after the enhancement.

Note that Figs. 3(b) and 4(a) to 4(c) illustrate a case where an absolute value of the threshold is equal regardless of whether the edge component is positive or negative, but the present invention is not limited thereto, and the absolute value of the threshold may be changed depending on whether the edge component is positive or negative.

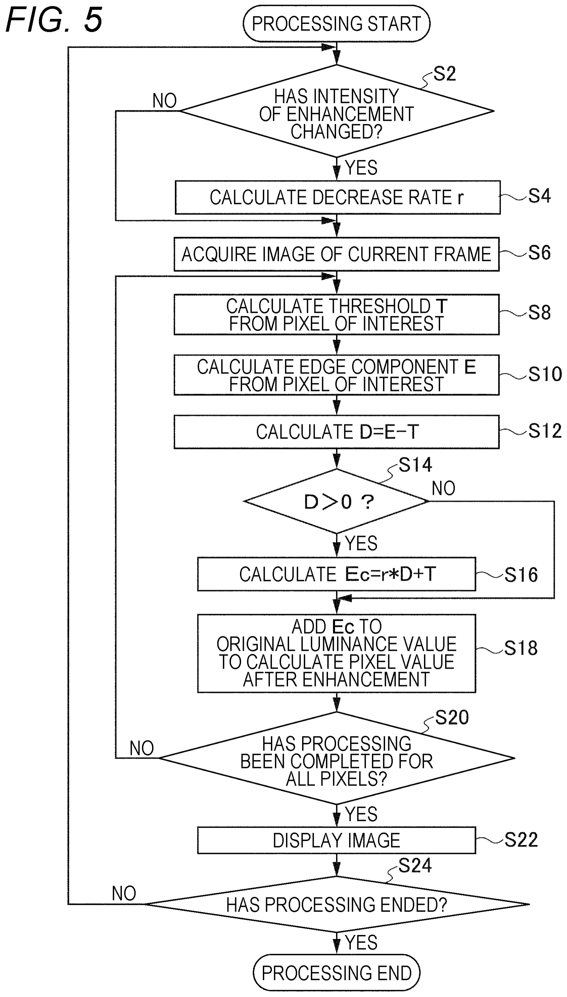

Next, an operation of the processor 20 of the present embodiment will be described with reference to Fig. 5.

The processor 20 sequentially monitors whether or not the intensity of edge enhancement has changed, and in a case where the intensity of edge enhancement has changed (step S2: YES), the processor 20 calculates the decrease rate r when the portion exceeding the threshold is decreased in the edge component detected from the input image based on the above Formula (1) (step S4). As described above, the intensity of edge enhancement is changed based on, for example, the operation input to the operation panel 26.

The processor 20 acquires an image signal (current frame) from the image input processing unit 22 in units of frames (step S6), and accumulates the image signal in the image memory 23. The enhancement calculation unit 27 performs the processing from steps S8 to S22 on the current frame.

The enhancement calculation unit 27 of the processor 20 sets each of pixels of the current frame as a pixel of interest and performs the processing from steps S8 to S18 on all the pixels (step S20: NO). Based on a luminance value of the pixel of interest, the enhancement calculation unit 27 calculates a threshold T with reference to the threshold setting data (step S8). The enhancement calculation unit 27 calculates an edge component E from the luminance values of the pixel of interest and the surrounding pixels (step S10). The enhancement calculation unit 27 calculates an edge component D (= E - T) of a portion exceeding the threshold T in the edge component E obtained in step S10 (step S12). Note that the edge component and the threshold may be positive or negative as illustrated in Fig. 3, but the threshold T and the edge component E referred to in this flowchart are absolute values.

In a case where D > 0 (step S14: YES), the portion exceeding the threshold T in the edge component E obtained in step S10 is substantially present, the edge component E calculated in step S10 is corrected. Specifically, Ec = r × D + T is calculated using the decrease rate r obtained in step S4, thereby calculating the corrected edge component Ec so as to decrease the portion exceeding the threshold T in the edge component E obtained in step S10 (step S16). Then, the enhancement calculation unit 27 adds the corrected edge component Ec to the luminance value of the pixel of interest to calculate a pixel value (RGB value) of the pixel of interest (step S18). As a result, the pixel value after the enhancement of the pixel of interest is obtained.

In a case where the processing has been completed for all the pixels of the current frame (step S20: YES), the processor 20 converts an image of the current frame to which the edge enhancement has been applied into a video format signal and displays the image on the monitor 30 (step S22).

In a case where the processing has not ended (step S24: NO), the processor 20 returns to step S2 to process the next frame.

As described above, according to the electronic endoscope system 1 of the present embodiment, the edge component is detected for each pixel of the captured image of the living tissue, and the detected edge component of each pixel is corrected with reference to the threshold setting data in which the threshold of the edge component in accordance with the luminance value is set. Furthermore, the edge enhancement processing is performed on each pixel based on the corrected edge component. Therefore, the edge component E is limited to be equal to or less than the threshold set in advance in accordance with the luminance value, so that the unnatural emphasis (edge undershoot) in which the whitened portion is bordered in black is less likely to occur in the image displayed on the monitor 30.

Preferably, in a case where the detected edge component exceeds the threshold indicated by the threshold setting data, the edge component is corrected based on the parameter set to decrease the portion exceeding the threshold in the edge component. At this time, the parameter is set such that the portion exceeding the threshold decreases as the intensity of enhancement of the edge enhancement processing increases. As a result, it is possible to appropriately emphasize the portion to be originally emphasized while suppressing the occurrence of the large undershoot.

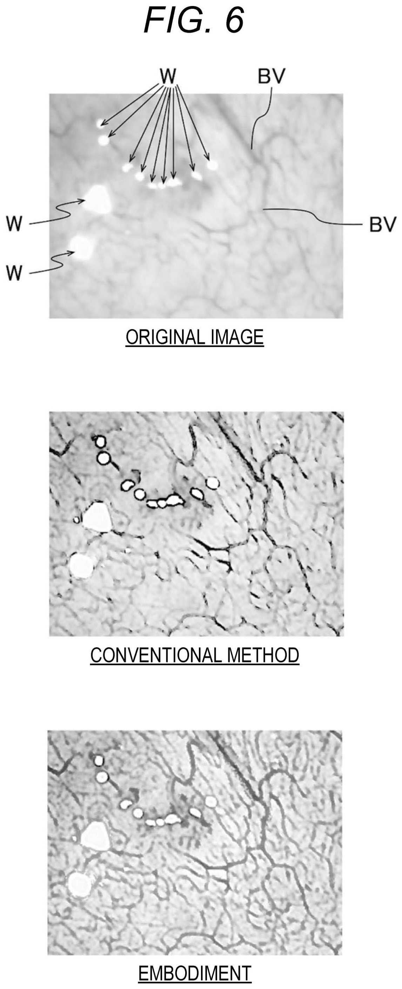

Referring to Fig. 6, a captured image (original image) of a living tissue as an example before enhancement, an image in a case where edge enhancement is performed on the original image by a conventional edge enhancement method (conventional method), and an image in a case where edge enhancement is performed on the original image by an edge enhancement method (embodiment) of the present embodiment are illustrated.

As illustrated in the original image, when the living tissue is captured, high-luminance whitened portions W are generated in addition to blood vessel portions BV by specular reflection of illumination light of an object covered with a mucous membrane such as a stomach or a large intestine. When the edge enhancement is performed on the whitened portions W by the conventional method, it is confirmed that unnatural emphasis (edge undershoot) in which the whitened portions are bordered in black occurs and the blood vessel portions BV are not sufficiently emphasized but blurred.

On the other hand, in the case of the embodiment, it can be seen that the edge undershoot is suppressed to an inconspicuous extent, and the blood vessel portions BV are appropriately emphasized and easily observed.

In one embodiment, the enhancement calculation unit 27 performs edge enhancement processing according to an edge enhancement processing method selected in accordance with an operation of the user from among a plurality of edge enhancement processing methods. In such a case, the plurality of edge enhancement processing methods are associated with different pieces of the threshold setting data, respectively. The edge component correction unit 273 corrects the edge component E with reference to the threshold setting data associated with the selected edge enhancement processing method. Therefore, the observer can switch the edge enhancement processing method such as a spatial filter by the input with respect to the operation panel 26 according to the purpose or preference, and the flexible operation according to the observer can be performed.

For example, a plurality of spatial filters may be prepared for the edge enhancement. Among them, there may be a spatial filter specialized for enhancing a portion with a higher frequency in the captured image, and there may be a spatial filter specialized for enhancing a portion with a lower frequency. In this case, when an applied spatial filter is switched, corresponding threshold setting data and the parameter or the function f (see Formula (1)) at the time of calculating the decrease rate r are changed. Such change processing is performed in a part corresponding to steps S2 to S4 in the flowchart of Fig. 5.

In one embodiment, the light source device 25 generates, as the illumination light, either first illumination light in a first wavelength band or second illumination light in a second wavelength band different from the first wavelength band. The first illumination light and the second illumination light are not limited as long as the first illumination light and the second illumination light have spectra different from each other. As an example, the first illumination light is normal light (white light or pseudo white light), and the second illumination light is special light (light of a wavelength band narrower than a wavelength band of the normal light). Since an observation image by the special light enables acquisition of an image different from an observation image by the normal light according to absorption characteristics of a living tissue, a certain characteristic portion of the living tissue can be emphasized and observed, and a lesion or the like of the living tissue can be easily found.

Since images obtained by the first illumination light and the second illumination light are different from each other, it is preferable that pieces of threshold setting data be associated with beams of illumination light, respectively. Then, the edge component correction unit 273 corrects the edge component E with reference to the threshold setting data associated with the illumination light generated by the light source device 25 out of the first illumination and the second illumination light. As a result, an appropriate threshold can be set in accordance with the illumination light. At that time, it is preferable to change the parameter or the function f (see Formula (1)) at the time of calculating the decrease rate r in accordance with the illumination light. In this case, the threshold setting data to be referred to and the parameter or the function f are changed in accordance with an operation input of the observer with respect to the operation panel 26 for selecting either the first illumination light or the second illumination light. Such change processing is performed in a part corresponding to steps S2 to S4 in the flowchart of Fig. 5.

In one embodiment, the plurality of edge enhancement processing methods are provided in the enhancement calculation unit 27, and the light source device 25 is configured to emit a plurality of beams of illumination light of different spectra. The edge enhancement processing method (spatial filter) is selected and the illumination light is selected in accordance with an operation of the observer. In this case, the threshold setting data and the parameter or the function f to be applied is preferably set to correspond to all combinations of the spatial filter and the illumination light.

While the processor for an electronic endoscope and the electronic endoscope system according to the present invention have been described above in detail, the processor for an electronic endoscope and the electronic endoscope system according to the present invention are not limited to the above-described embodiment, and can be modified or altered in various ways without departing from the spirit of the present invention.

The present invention relates to a patent application of Japanese Patent Application No. 2023-2336 filed with the Japan Patent Office on January 11, 2023, the entire contents of which are incorporated herein by reference.

**Claims**

1. A processor for an electronic endoscope that acquires a captured image of a living tissue and performs image processing, the processor comprising:

   an edge detection unit that detects an edge component for each of pixels of the captured image of the living tissue;
   an edge component correction unit that corrects the edge component of each of the pixels detected by the edge detection unit with reference to threshold setting data in which a threshold of the edge component in accordance with a luminance value is set; and
   an enhancement processing unit that performs contour enhancement processing on the captured image based on the edge component corrected by the edge component correction unit.

2. The processor for an electronic endoscope according to claim 1, wherein
   in the threshold setting data, the threshold is set in such a manner that the threshold of the edge component increases as the luminance value increases.

3. The processor for an electronic endoscope according to claim 1 or 2, wherein

   when the edge component detected by the edge detection unit exceeds the threshold, the edge component correction unit corrects the edge component based on a parameter set to decrease a portion exceeding the threshold in the edge component, and
   the parameter is set in such a manner that the portion exceeding the threshold decreases as an intensity of enhancement of the contour enhancement processing increases.

4. The processor for an electronic endoscope according to claim 3, further comprising

   an intensity changing unit that changes the intensity of enhancement of the contour enhancement processing in accordance with an operation of a user, wherein
   when the intensity of enhancement of the contour enhancement processing is changed, the edge component correction unit adjusts the parameter based on the changed intensity.

5. The processor for an electronic endoscope according to claim 1 or 2, wherein

   the enhancement processing unit performs the contour enhancement processing according to a contour enhancement processing method selected in accordance with an operation of a user from among a plurality of the contour enhancement processing methods,
   the plurality of contour enhancement processing methods are associated with different pieces of the threshold setting data, respectively, and
   the edge component correction unit corrects the edge component with reference to the threshold setting data associated with the selected contour enhancement processing method.

6. The processor for an electronic endoscope according to claim 3, wherein

   the enhancement processing unit performs the contour enhancement processing according to a contour enhancement processing method selected in accordance with an operation of a user from among a plurality of the contour enhancement processing methods,
   the plurality of contour enhancement processing methods are associated with a plurality of the parameters different from each other, respectively, and
   the edge component correction unit corrects the edge component with reference to the parameter associated with the selected contour enhancement processing method.

7. The processor for an electronic endoscope according to claim 1 or 2, further comprising

a light source unit that generates, as illumination light for illuminating the living tissue, either first illumination light in a first wavelength band or second illumination light in a second wavelength band different from the first wavelength band, wherein

the first illumination light and the second illumination light are associated with different pieces of the threshold setting data, and

the edge component correction unit corrects the edge component with reference to the threshold setting data associated with the illumination light generated by the light source unit out of the first illumination and the second illumination light.

8. The processor for an electronic endoscope according to claim 3, further comprising

a light source unit that generates, as illumination light for illuminating the living tissue, either first illumination light in a first wavelength band or second illumination light in a second wavelength band different from the first wavelength band, wherein

the first illumination light and the second illumination light are associated with a plurality of the parameters different from each other, respectively, and

the edge component correction unit corrects the edge component with reference to the parameter associated with the illumination light generated by the light source unit out of the first illumination and the second illumination light.

9. An endoscope system comprising:

the processor for an endoscope according to any one of claims 1 to 8; and

an endoscope connected to the processor for an endoscope and comprising an image sensor that captures the living tissue.

## FIG. 1

EP 4 649 882 A1

## FIG. 2

# FIG. 3

EDGE COMPONENT

A

C

B

LUMINANCE VALUE

(a)

EDGE COMPONENT

DECREASE

THRESHOLD

THRESHOLD

DECREASE

LUMINANCE VALUE

(b)

# FIG. 4

*FIG. 5*

```
                    ( PROCESSING START )
                            │
                            ▼
        NO                                          S2
      ┌──────◄   HAS INTENSITY
      │          OF ENHANCEMENT
      │            CHANGED?
      │               │ YES
      │               ▼
      │      CALCULATE DECREASE RATE r          S4
      │               │
      └───────────────┤
                      ▼
           ACQUIRE IMAGE OF CURRENT FRAME        S6
                      │
      ┌───────────────┤
      │               ▼
      │      CALCULATE THRESHOLD T                S8
      │      FROM PIXEL OF INTEREST
      │               │
      │               ▼
      │      CALCULATE EDGE COMPONENT E          S10
      │      FROM PIXEL OF INTEREST
      │               │
      │               ▼
      │         CALCULATE D=E−T                  S12
      │               │
      │               ▼                  S14
      │                              NO
      │            D>0 ? ────────────┐
      │               │ YES          │
      │               ▼              │
      │      CALCULATE  Ec=r*D+T      │    S16
      │               │              │
      │               ◄──────────────┘
      │               ▼
      │            ADD Ec TO
      │      ORIGINAL LUMINANCE VALUE            S18
      │      TO CALCULATE PIXEL VALUE
      │         AFTER ENHANCEMENT
      │               │
      │               ▼                  S20
      │  NO        HAS PROCESSING
      └──◄       BEEN COMPLETED FOR
                     ALL PIXELS?
                      │ YES
                      ▼
              DISPLAY IMAGE                      S22
                      │
                      ▼                   S24
     NO        HAS PROCESSING ENDED?
   ◄───
                      │ YES
                      ▼
              ( PROCESSING END )
```

# FIG. 6

ORIGINAL IMAGE

CONVENTIONAL METHOD

EMBODIMENT

## INTERNATIONAL SEARCH REPORT

| International application No. |
| --- |
| **PCT/JP2023/044619** |

**A.    CLASSIFICATION OF SUBJECT MATTER**

*A61B 1/045*(2006.01)i
FI:    A61B1/045 610

According to International Patent Classification (IPC) or to both national classification and IPC

**B.    FIELDS SEARCHED**

Minimum documentation searched (classification system followed by classification symbols)

A61B1/045

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2009-021905 A (HOYA CORPORATION) 29 January 2009 (2009-01-29) paragraphs [0013]-[0038] | 1-2, 9 |
| A | | 3-8 |
| A | JP 2014-117412 A (FUJIFILM CORPORATION) 30 June 2014 (2014-06-30) entire text, all drawings | 1-9 |
| A | JP 2014-144034 A (OLYMPUS CORPORATION) 14 August 2014 (2014-08-14) entire text, all drawings | 1-9 |
| A | JP 2011-254214 A (HOYA CORPORATION) 15 December 2011 (2011-12-15) entire text, all drawings | 1-9 |
| A | JP 6-046293 A (CASIO COMPUTER CO., LTD.) 18 February 1994 (1994-02-18) entire text, all drawings | 1-9 |

☐ Further documents are listed in the continuation of Box C.       ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **15 February 2024** | **05 March 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2023/044619**

| Patent document cited in search report | | | Publication date (day/month/year) | Patent family member(s) | | | Publication date (day/month/year) |
|---|---|---|---|---|---|---|---|
| JP | 2009-021905 | A | 29 January 2009 | US | 2009/0016635 | A1 | |
| | | | | paragraphs [0019]-[0053] | | | |
| | | | | DE | 102008032989 | A1 | |
| JP | 2014-117412 | A | 30 June 2014 | EP | 2743887 | A2 | |
| | | | | entire text, all drawings | | | |
| JP | 2014-144034 | A | 14 August 2014 | US | 2015/0294463 | A1 | |
| | | | | entire text, all drawings | | | |
| | | | | WO | 2014/115371 | A1 | |
| JP | 2011-254214 | A | 15 December 2011 | (Family: none) | | | |
| JP | 6-046293 | A | 18 February 1994 | (Family: none) | | | |

Form PCT/ISA/210 (patent family annex) (July 2022)

**REFERENCES CITED IN THE DESCRIPTION**

**Patent documents cited in the description**

- JP 2009021905 A
- JP 2023002336 A